# EUROPÄISCHE PATENTSCHRIFT

(11) **EP 0 790 046 B1**
(45) Veröffentlichungstag und Bekanntmachung des Hinweises auf die Patenterteilung: **16.05.2001**
(21) Anmeldenummer: 97101023.6
(22) Anmeldetag: 23.01.1997
(51) Int. Cl.: A61F 5/01

(54) **Gelenkstütze, insbesondere Kniegelenkstütze**
Joint support, particularly for the knee
Support de l'articulation, en particulier du genou

(30) Priorität: 19.02.1996 DE 19606092
(43) Veröffentlichungstag der Anmeldung: 20.08.1997
(73) Patentinhaber: Albrecht GmbH, 83115 Neubeuern (DE)
(72) Erfinder: Opahle, Hans Georg, 83024 Rosenheim (DE); Albrecht, Erich, 83024 Rosenheim (DE)
(74) Vertreter: Bauer, Friedrich, Dipl.-Ing.

(56) Entgegenhaltungen:
- WO-A-95/01141
- US-A- 2 883 982
- US-A- 4 463 751
- US-A- 5 286 250
- US-E- R E33 621

## Beschreibung

Die Erfindung betrifft eine Gelenkstütze, insbesondere Kniegelenkstütze, zur Verhinderung einer Torsion eines distalen Körperglieds gegenüber einem damit gelenkig verbundenen proximalen Körperglied, gemäß dem Oberbegriff des Patentanspruches 1.

Gelenkstützen dieser Art sind als Knieorthesen bekannt, die beispielsweise nach Kreuzbandoperationen angelegt werden und eine Drehbewegung des Unterschenkels relativ zum Oberschenkel verhindern sollen. Die beidseits des Knies entlanggeführten Schienen sind in Höhe des Kniegelenks gelenkig verbunden und werden zu ihren freien Enden hin am Ober- bzw. Unterschenkel festgegurtet. Das Drehgelenk erlaubt hierbei ein Abknicken bzw. Strecken des Beins über einen bestimmten, festlegbaren Schwenkbereich.

Es ist darüber hinaus bekannt, den Schwenkbereich der Schienen und damit denjenigen des Kniegelenks in vorbestimmter Weise und variabel zu beschränken. Dies kann beispielsweise durch das Einlegen verschieden dimensionierter Keile zwischen dem Anschlag einer Schiene und dem Gegenanschlag der damit verbundenen weiteren Schiene erfolgen. Ferner ist es bekannt, einen Anschlagstift je nach gewünschtem Schwenkbereich in verschiedene Bohrungen einzusetzen, welche an einer der miteinander verbundenen Schienen vorgesehen sind.

Nachteilig ist bei diesen bekannten Gelenkstützen, daß eine Vergrößerung oder Verkleinerung des freien Schwenkbereichs nur in relativ großen Winkelschritten möglich ist. Beispielsweise kann bei einer üblichen Knieorthese die Schwenkbereichsbegrenzung in Extensionsrichtung, d.h. in Streckrichtung der Gliedmaßen, zwischen 0° und 45° nur in vier verschiedenen Positionen, d.h. in 15°-Schritten, eingestellt werden. Die 0°-Position entspricht dabei der vollkommen geraden Streckung des Unterschenkels bezüglich des Oberschenkels.

Aus der US 4 463 751 ist eine Gelenkstütze gemäß dem Oberbegriff des Patentanspruches 1 bekannt, bei welcher zwei innenliegende, kreisförmige Einstellscheiben mit Aussparungen zur Extensions- bzw. Flexionsbegrenzung der distalen Schiene verwendet werden. Durch die Aussparungen sind Stifte hindurchgeführt, die sich innerhalb der Aussparungen zusammen mit der distalen Schiene bewegen und in den entsprechenden Endstellungen an Anschlagflächen der Aussparungen anschlagen.

Weiterhin ist aus der US 3 732 862 eine Fußgelenkstütze bekannt, die ein Anheben der Fußspitze ermöglichen, deren Absenken unterhalb einer bestimmten Grundstellung jedoch verhindern soll. Diese bekannte Fußstütze weist ein drehbar gelagertes Schwenkteil mit einem unteren Anschlag und einer Mehrzahl oberer Durchgangslöcher auf, um dieses Schwenkteil mittels eines Befestigungsstiftes in unterschiedlichen Neigungswinkeln bezüglich einer Unterschenkelschiene zu halten.

Aus der US 2 883 982 ist eine Kniegelenkstütze bekannt, bei welcher zwei aufeinanderliegende Flächen der distalen und proximalen Schiene zahnradartig ausgebildete und aufeinander abwälzende Flächen aufweisen. Die Extensionslage wird dort mittels eines Zugankers begrenzt, der mit einem Ende innerhalb einer kulissenartigen Aussparung der proximalen Schiene und mit seinem anderen Ende schwenkbar an der distalen Schiene befestigt ist. Diese letztgenannten Schienen sind jedoch kompliziert aufgebaut, kostspielig und umständlich handhabbar. Außerdem sind sie nicht zum Dehnen des betreffenden Gelenkes geeignet, falls dessen Beweglichkeit aufgrund von Bänderoperationen, Unfällen, Entzündungen etc. eingeschränkt ist und Gelenkskapseln und/oder Bindegewebe ein Streck- oder Beugedefizit aufweisen.

Der Erfindung liegt daher die Aufgabe zugrunde, eine Gelenkstütze der eingangs genannten Art zu schaffen, die bei möglichst einfachem Aufbau eine größtmögliche Variabilität hinsichtlich der Einstellung der Schwenkbereichsbegrenzungen sowie eine sehr genaue und einfache Einstellung ermöglicht.

Diese Aufgabe wird erfindungsgemäß durch die Merkmale des Anspruches 1 gelöst. Vorteilhafte Ausführungsformen der Erfindung sind in den weiteren Ansprüchen beschrieben.

Erfindungsgemäß ist das Anschlagelement in vorbestimmten, unterschiedlichen Positionen an einem schwenkachsennahen Gelenkabschnitt einer Schiene befestigbar. Weiterhin besteht das Gegenanschlagverstellelement aus einem Schwenkhebel mit einem als Handhabungsabschnitt ausgebildeten Hebelarm, der sich über den schwenkachsennahen Gelenkabschnitt hinaus erstreckt und mittels eines innerhalb einer Langlocheinrichtung verschiebbaren Führungs- und Feststellelementes an der dem Gegenanschlagverstellelement zugeordneten Schiene festlegbar ist.

Durch diese erfindungsgemäße Ausgestaltung wird eine zusätzliche Verstellmöglichkeit für die Schwenkbereichsgrenzen geschaffen, da nicht nur das Anschlagelement in verschiedenen Positionen auf einer der Schienen befestigt werden kann, sondern auch die Position des Gegenanschlags relativ zur anderen Schiene. Dies bedeutet mit anderen Worten, daß dann, wenn der Gegenanschlag am Anschlagelement anliegt, eine weitere Veränderung der Schwenkbegrenzung dadurch vorgenommen werden kann, daß die das Gegenanschlagverstellelement führende Schiene nochmals relativ zum Gegenanschlagverstellelement geschwenkt wird. Es ergeben sich hierdurch zusätzliche Verstellmöglichkeiten, wobei die Schwenkbereichsbegrenzung der distalen Schiene gegenüber der proximalen Schiene auf sehr genaue, stufenlose Weise verändert und Gelenke und Bindegewebe gegebenenfalls bis zu einer sehr genau einstellbaren Grenze gequengelt, d.h. gedehnt werden können. Die zusätzliche Verstellung kann hierbei auf sehr einfache Weise erfolgen, da der Handhabungsteil des Gegenanschlagverstellelements gut zugänglich ist und nach Lösen des Führungs- und Verstellelementes manuell bezüglich der zugehörigen Schiene verschwenkt werden kann.

Vorteilhafterweise weisen der Handhabungsabschnitt des Gegenanschlagverstellelements und die diesem zugeordnete Schiene jeweils ein Langloch auf, deren Längsachsen sich in einem bestimmten Winkel schneiden. Hierbei ist das Führungs- und Feststellelement durch die beiden Langlöcher in deren Überschneidungsbereich hindurchgeführt, so daß eine Verschiebung des Führungs- und Feststellelementes zwangsweise eine Relativverschiebung zwischen dem Gegenanschlagverstellelement und der Schiene bewirkt. Bei dieser Ausführungsform gleitet das Führungs- und Feststellelement beim Verstellvorgang innerhalb der Längsschlitze, wobei ein relativ langer Verschiebeweg des Führungs- und Feststellelements eine relativ kleine Relativverschwenkung zwischen Gegenanschlagverstellelement und Schiene ergibt. Auf diese Weise läßt sich die zusätzliche Winkelverstellung stufenlos sowie auf sehr genaue und feinfühlige Weise vornehmen. Weiterhin werden in dem Fall, daß der Patient die Gelenkschiene über den maximalen Schwenkbereich hinaus zu bewegen versucht, ein Großteil der Kräfte über die seitlichen Begrenzungswände der Längsschlitze aufgenommen, so daß ein unbeabsichtigtes Verschieben des Führungs- und Feststellelements auch bei geringeren Festklemmkräften verhindert werden kann. Aufgrund der einfachen stufenlosen Verstellung der Schiene relativ zum Gegenanschlagverstellelement lassen sich die Gelenke auf besonders feinfühlige Weise bis in die gewünschte Grenzposition quengeln.

Eine besonders vorteilhafte Ausgestaltung der Erfindung zeichnet sich dadurch aus, daß der Verstellbereich der Schiene gegenüber dem Gegenanschlagverstellelement 0° bis -20°, insbesondere 0° bis -15° in Extensionsrichtung, d.h. in Streckrichtung der Körperglieder, bezüglich der durch den Anschlag festgelegten Winkelposition beträgt. Dies bedeutet, daß beispielsweise dann, wenn sich das Anschlagelement in seiner 0°-Extensionsbegrenzungsposition befindet, die maximale Extension der Gelenkstütze nicht 0° beträgt, was einem gerade gestreckten Bein entspräche, sondern eine weitere Winkelveränderung des Gegenanschlags in Richtung Extension ermöglicht wird, so daß eine Überstreckung des Unterschenkels bis maximal -15° bzw. - 20° möglich ist. Obwohl derart starke Überstreckungen nur in wenigen Ausnahmefällen erwünscht sein dürften, wird eine geringe Überstreckung vermehrt dann angewandt, wenn beispielsweise das andere, gesunde Gelenk auch bereits eine derartige Überstreckung aufweist.

Die Erfindung wird nachfolgend anhand von Zeichnungen beispielsweise näher erläutert. In diesen zeigen:
- Figur 1 :: eine Seitenansicht der erfindungsgemäßen Gelenkstütze;
- Figur 2 :: eine perspektivische Ansicht des Gelenkbereich der Gelenkstütze von Figur 1;
- Figur 3 :: eine schematische Explosionsdarstellung der Einzelteile der Gelenkstütze von Figur 1;
- Figuren 4A und 4B :: eine Seitenansicht der Gelenkstütze von Figur 1 mit und ohne Gewindescheibe in der 0°-Extensionsbegrenzungsposition;
- Figuren 5A und 5B :: eine Seitenansicht der Gelenkstütze von Figur 1 mit und ohne Gewindescheibe in der 60°-Flexionsbegrenzungsposition;
- Figuren 6A und 6B :: eine Seitenansicht der Gelenkstütze von Figur 1 mit und ohne Gewindescheibe in der -15°-Extensionsbegrenzungsposition (Überstreckungsposition); und
- Figur 7 :: eine schematische Vorderansicht der gesamten Gelenkstütze gemäß der Erfindung.

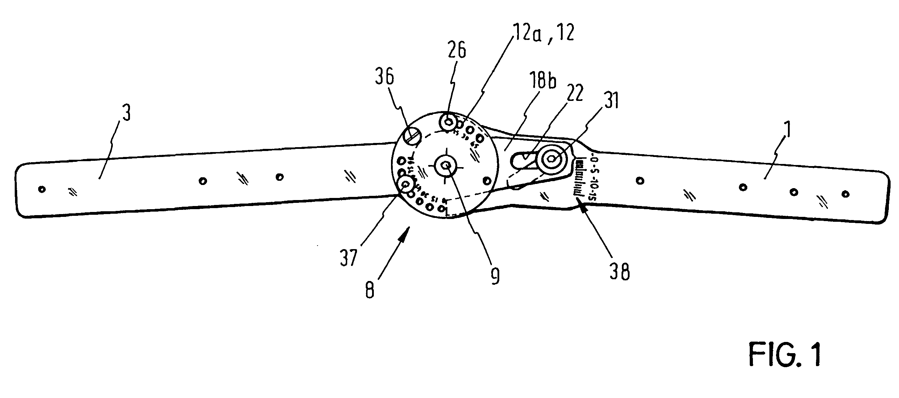

Im folgenden wird anhand der Figuren 1 bis 7 die erfindungsgemäße Gelenkstütze beschrieben, wobei im Normalfall zwei Gelenkstützen verwendet werden, die sich auf gegenüberliegenden Seiten des Gelenks befinden. Die auf der gegenüberliegenden Seite des Gelenks anzuordnende Gelenkstütze ist spiegelbildlich ausgebildet. Befestigungsgurte zur Befestigung der Gelenkstütze an einem proximalen bzw. distalen Körperglied sind in den Figuren 1 bis 6B der Übersichtlichkeit halber weggelassen. Die erfindungsgemäße Gelenkstütze wird anhand des Beispiels einer Knieorthese beschrieben, könnte jedoch auch für andere Gelenke, beispielsweise für das Ellbogengelenk, Anwendung finden.

Wie aus den Figuren 1 bis 3 ersichtlich, besteht die erfindungsgemäße Gelenkstütze aus einer distalen Schiene 1, welche mittels entsprechender, nicht gezeigter Gurte am Unterschenkel befestigt werden kann, sowie einer proximalen Schiene 3, welche ebenfalls mittels nicht gezeigter Gurte am Oberschenkel befestigbar und über ein Drehgelenk 8 gelenkig mit der distalen Schiene 1 verbunden ist. Die Schwenkachse ist hierbei mit dem Bezugszeichen 9 bezeichnet.

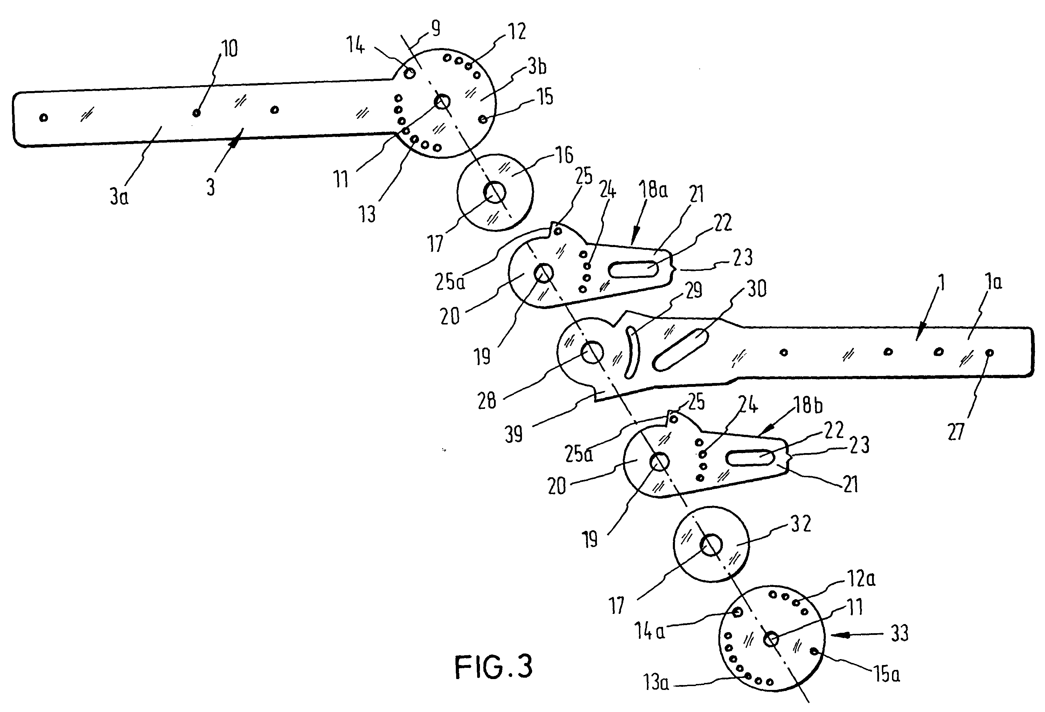

Die erfindungsgemäße Gelenkstütze ist in Figur 3 zur Veranschaulichung der wesentlichen Einzelteile explosionsartig dargestellt. Wie ersichtlich, besteht die proximale Schiene 3 aus einem länglichen Befestigungsabschnitt 3a und einem kreisscheibenförmigen Gelenkabschnitt 3b, der an einem Ende des Befestigungsabschnittes 3a einstückig angeformt ist. Im Befestigungsabschnitt 3a sind mehrere Durchgangslöcher 10 zur Befestigung der nicht dargestellten Gurte vorgesehen.

Der kreisscheibenförmige Gelenkabschnitt 3b weist in seinem Zentrum ein Loch 11 zum Durchtritt einer nicht dargestellten Drehgelenkachse auf. In der Nähe des Außenumfangs sind weiterhin vier Extensionsbegrenzungslöcher 12 vorgesehen, die in Umfangsrichtung nebeneinanderliegend angeordnet sind. Zwei benachbarte Extensionsbegrenzungslöcher 12 schließen hierbei miteinander jeweils einen Winkel von 15° ein.

Auf der anderen Seite des mittigen Lochs 11 sind insgesamt sieben Flexionsbegrenzungslöcher 13 vorgesehen, die ebenfalls in der Nähe des Außenumfangs des Gelenkabschnitts 3b und in Umfangsrichtung nebeneinanderliegend angeordnet sind. Zwei nebeneinanderliegende Flexionsbegrenzungslöcher 13 sind wiederum mit einem Winkel von jeweils 15° voneinander beabstandet.

Zwischen den Extensionsbegrenzungslöchern 12 und den Flexionsbegrenzungslöchern 13 ist weiterhin etwa im gleichen Abstand zur Schwenkachse 9 ein Befestigungsloch 14 zum Hindurchführen einer nicht gezeigten Befestigungsschraube vorgesehen. Ein Arretierloch 15 dient zum Hindurchführen einer nicht gezeigten Arretierschraube, falls die Schwenkbewegung der proximalen Schiene 3 gegenüber der distalen Schiene 1 vollständig blockiert werden soll. Sämtliche Löcher 12, 13, 14 und 15 sind als Durchgangslöcher ausgeführt und befinden sich in etwa auf einer gemeinsamen Kreisbahn um die Schwenkachse 9.

Die proximale Schiene 3 stellt das seitlich am weitesten innen liegende Teil der Gelenkstütze dar, das somit, mit einer entsprechenden Polsterung versehen, direkt am Oberschenkel des Patienten anliegt.

Wie aus Figur 3 weiter ersichtlich, schließt an den Gelenkabschnitt 3b der proximalen Schiene 3 in Richtung nach außen eine kreisförmige Reibungsverminderungsscheibe 16 an, die beispielsweise aus einem dünnen Kunststoffblatt bestehen kann. Die Reibungsverminderungsscheibe 16 weist eine mittige Durchgangsöffnung 17 zum Durchtritt der Schwenkachse auf.

In Richtung nach außen folgt eine Hälfte 18a eines Gegenanschlagverstellelements 18, die an der Innenseite der distalen Schiene 1 anliegt. Die andere Hälfte 18b des Gegenanschlagverstellelements 18 ist bis auf eine später noch näher beschriebene, spiegelbildlich angeordnete Eindellung identisch zur Hälfte 18a und liegt auf der gegenüberliegenden Seite der distalen Schiene 1 an dieser an.

Jede Gegenanschlagverstellelementhälfte 18a, 18b besteht aus einem dünnen, jedoch festen Stahlblechteil, insbesondere aus Edelstahl, und ist schwenkbar um die Schwenkachse 9 herum gelagert. Hierzu weisen die Gegenanschlagverstellelementhälften 18a, 18b eine Durchgangsöffnung 19 auf, durch welche die Drehgelenkachse hindurchgeführt werden kann. Das proximale Ende der Gegenanschlagverstellelementhälften 18a, 18b ist in der Form eines halbkreisförmigen Abschnitts 20 ausgebildet, wobei der Radius dieses Halbkreises kleiner ist als der Radius des Kreisbogens, auf dem die Extensionsbegrenzungslöcher 12 und die Flexionsbegrenzungslöcher 13 der proximalen Schiene 3 liegen. Diese Löcher 12, 13 werden somit vom halbkreisförmigen Abschnitt 20 der Gegenanschlagverstellelementhälften 18a, 18b nicht abgedeckt. Zweckmäßigerweise ist der Radius des halbkreisförmigen Abschnitts 20 gleich demjenigen der Reibungsverminderungsscheibe 16.

An den halbkreisförmigen Abschnitt 20 der Gegenanschlagverstellelementhälften 18a, 18b schließt sich ein länglicher Handhabungsabschnitt 21 an, der sich über den Gelenkabschnitt 3b der proximalen Schiene 3 hinaus etwa in Längsrichtung der distalen Schiene 1 erstreckt. In diesem Handhabungsabschnitt 21 befindet sich ein in Längsrichtung der Gegenanschlagverstellelementhälften 18a, 18b ausgerichtetes Langloch 22, dessen Längsachse die Schwenkachse 9 schneidet. Die Länge des Langlochs 22 beträgt etwa drei bis viermal seiner Breite. Am distalen Ende des Handhabungsabschnitts 21 ist eine als Zeiger wirkende Spitze 23 vorgesehen, die in der Längsachse der Gegenanschlagverstellelementhälften 18a, 18b liegt.

Zwischen dem Langloch 22 und der Durchgangsöffnung 19 sind auf einem Kreisbogen um die Schwenkachse 9 herum vier Arretierungslöcher 24 vorgesehen, die voneinander jeweils einen Winkelabstand von 15° aufweisen. Der Radius dieses Kreisbogens entspricht dem radialen Abstand des Arretierlochs 15 der proximalen Schiene 3 von der Schwenkachse 9, so daß das Arretierungsloch 15 in bestimmten Winkellagen mit den Arretierungslöchern 24 zur Deckung gebracht und eine nicht dargestellte Arretierschranke hindurchgeführt werden kann.

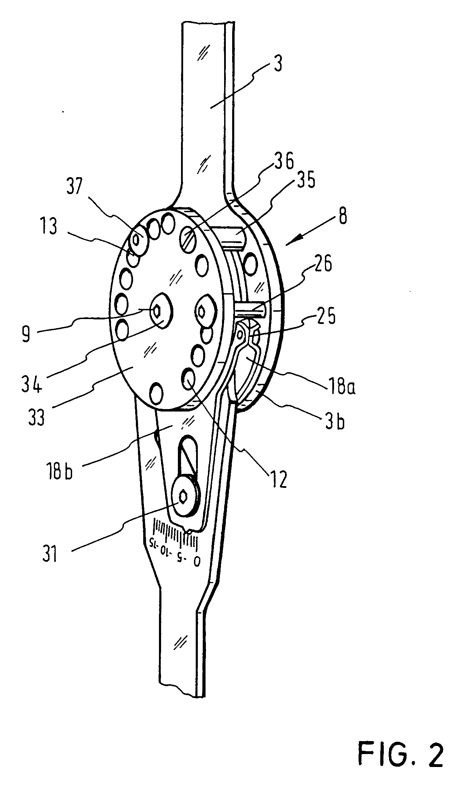

An den halbkreisförmigen Abschnitt 20 der Gegenanschlagverstellelementhälften 18a, 18b schließt sich ein in Figur 3 nach oben zeigender Gegenanschlag 25 in Form einer radial über den halbkreisförmigen Abschnitt 20 vorstehenden Nase an. Diese Nase erstreckt sich in radialer Richtung über die Extensionsbegrenzungslöcher 12 hinaus nach außen, so daß eine stirnseitige Kontaktfläche 25a des Gegenanschlags 25 an ein stiftförmiges Anschlagelement 26 (Figur 3) anschlagen kann, das durch eines der Extensionsbegrenzungslöcher 12 hindurchgeführt ist. Wie aus Figur 2 ersichtlich, können die Nasen 25 der beiden Gegenanschlagverstellelementhälften 18a, 18b derart zueinander hin eingedellt und mittels eines Niets aneinander befestigt werden, daß sie sich radial außerhalb der distalen Schiene 1 berühren und eine entsprechende Querverbindung geschaffen wird. Alternativ wäre es jedoch auch ohne weiteres möglich, zwischen den Nasen 25 der beiden Gegenanschlagverstellelementhälften 18a, 18b eine Abstandshülse oder einen Abstandstift vorzusehen, um eine entsprechende gegenseite Halterung in diesem Bereich zu schaffen.

Wie weiterhin besonders deutlich aus Figur 3 ersichtlich ist, weist die distale Schiene 1 in ähnlicher Weise wie die proximale Schiene 3 einen länglichen Befestigungsabschnitt 1a auf, in welchem insgesamt vier Löcher 27 zur Befestigung nicht dargestellter Gurte vorgesehen sind. Am proximalen Ende ist eine Durchgangsöffnung 28 vorgesehen, durch welche die Drehgelenkachse hindurchgeführt werden kann, so daß die distale Schiene 1 um die Schwenkachse 9 herum geschwenkt werden kann. Das proximale Ende ist wiederum etwa halbkreisförmig ausgebildet, wobei sich der Kreisbogen über etwas mehr als 180°, beispielsweise 220°, erstrecken kann. Der Radius dieses halbkreisförmigen Abschnitts entspricht wiederum demjenigen des halbkreisförmigen Abschnitts 20 der Gegenanschlagverstellelementhälften 18a, 18b und dem Radius der Reibungsverminderungsscheibe 16.

Auf einem Kreisbogen um die Schwenkachse 9 herum ist ein bogenförmiger Schlitz 29 vorgesehen. Sind die beiden Gegenanschlagverstellelementhälften 18a, 18b derart zur distalen Schiene 1 ausgerichtet, daß die Längsachsen dieser Teile parallel zueinander sind, überdeckt dieser Schlitz 29 die Arretierungslöcher 24 der Gegenanschlagverstellelementhälften 18a, 18b vollständig, so daß die bereits erwähnte, nicht dargestellte Arretierschraube zur Schwenkblockade der distalen Schiene 1 gegenüber der proximalen Schiene 3 hindurchgeführt werden kann.

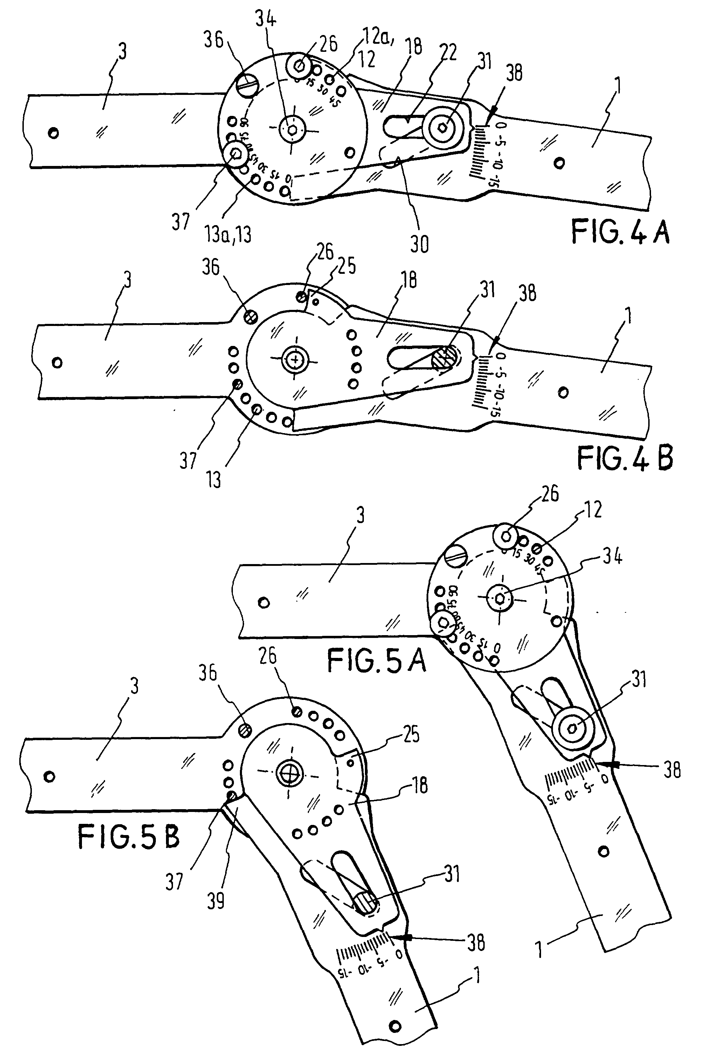

Zwischen dem bogenförmigen Schlitz 29 und dem Befestigungsabschnitt 1a der distalen Schiene 1 ist weiterhin ein schräg angeordnetes Langloch 30 vorgesehen, dessen Längsachse mit der Längsachse der distalen Schiene 1 im gezeigten Ausführungsbeispiel einen Winkel von 34° einschließt. Die Länge des Langloches 30 kann geringfügig größer sein als diejenige des Langlochs 22 der Gegenanschlagverstellelementhälften 18a, 18b, während seine Breite zweckmäßigerweise gleich ist. Die Anordnung und Länge der Langlöcher 22, 30 ist derart getroffen, daß sich ein in den Figuren 4A bis 6B näher gezeigtes Führungs- und Feststellelement 31, das durch die Langlöcher 22, 30 hindurchgeführt wird, an den beiden Verschwenkgrenzen des Gegenanschlagverstellelements 18 relativ zur distalen Schiene 1 an den beiden Enden der Langlöcher 22, 30 befindet.

Wie aus Figur 3 ersichtlich, schließt sich an der Außenseite der äußeren Gegenanschlagverstellelementhälfte 18b eine weitere Reibungsverminderungsscheibe 32 an, die in gleicher Weise wie die Reibungsverminderungsscheibe 16 ausgebildet ist und ebenso eine Durchgangsöffnung 17 zum Durchtritt der Drehgelenkachse aufweist.

Den seitlich äußeren Abschluß der Gelenkstütze bildet eine in Figur 3 lediglich schematisch dargestellte Gewindescheibe 33 mit Extensionsbegrenzungslöchern 12a, Flexionsbegrenzungslöchern 13a, einem Befestigungsloch 14a und einem Arretierungsloch 15a, welche den entsprechenden Löchern 12, 13, 14 bzw. 15 des kreisförmigen Gelenkabschnitts 3b der proximalen Schiene 3 fluchtend gegenüberliegen. Die Anzahl und Anordnung dieser Löcher 12a, 13a, 14a und 15a relativ zur Schwenkachse 9 ist daher identisch zu derjenigen der Löcher 12, 13, 14 bzw. 15. Die Löcher 12a, 13a, 14a und 15a sind jedoch mit einem Innengewinde versehen, um Schrauben bzw. Stifte einschrauben zu können, die im kopfnahen Schaftbereich ein Gewinde aufweisen. Weiterhin weist die vollständig als Kreisscheibe ausgebildete Gewindescheibe 33 den gleichen Radius und die gleiche mittige Durchgangsöffnung 11 zum Durchführen der Drehgelenkachse wie der kreisförmige Gelenkabschnitt 3b der proximalen Schiene 3 auf.

Die nicht dargestellte Drehgelenkachse kann aus einem hülsenförmigen Teil mit einer axialen, durchgehenden Gewindebohrung bestehen, in die, nach dem Zusammensetzen der in Figur 3 gezeigten Teile, zwei Halteschrauben 34 (Figur 2) eingeschraubt werden. Um die gewünschte beabstandete, parallele Anordnung der Gewindescheibe 33 zum kreisförmigen Gelenkabschnitt 3b der proximalen Schiene 3 aufrecht zu erhalten, ist weiterhin eine Distanzhülse 35 mit einer durchgehenden axialen Gewindebohrung vorgesehen, die fluchtend zu den Befestigungslöchern 14, 14a des Gelenkabschnitts 3b bzw. der Gewindescheibe 33 angeordnet wird. Entsprechende, von beiden Seiten in die Befestigungslöcher 14, 14a eingeführte Befestigungsschrauben 36 können damit mit der Distanzhülse 35 verschraubt werden.

Wie weiterhin aus Figur 2 ersichtlich, ist bei dem dargestellten Ausführungsbeispiel das stiftförmige Extensionsanschlagelement 26 in die beiden gegenüberliegenden, obersten Extensionsbegrenzungslöcher 12, 12a eingesetzt, wobei es über einen kurzen, an den Stiftkopf anschließenden Gewindeabschnitt mit der Extensionsbegrenzungsgewindebohrung 12a verschraubt ist. Weiterhin ist ein Flexionsanschlagelement 37 in zwei gegenüberliegende Flexionsbegrenzungslöcher 13, 13a eingesetzt, so daß eine Flexion der Gelenkstütze bis zu 60° ermöglicht wird, wie nachfolgend noch näher beschrieben wird.

Im folgenden wird anhand der Figuren 4A bis 6B die Funktionsweise der erfindungsgemäßen Gelenkstütze näher erläutert. Die entsprechenden, mit A bzw. B bezeichneten Figuren sind jeweils in der gleichen Stellung und von der gleichen Seite her abgebildet, unterscheiden sich jedoch darin, daß die mit B gekennzeichneten Zeichnungen der besseren Übersichtlichkeit halber ohne die Gewindescheibe 33 und ohne Reibungsverminderungsscheibe 33 dargestellt sind. Weiterhin ist dort das Führungs- und Feststellelement 31 im Schnitt dargestellt.

In sämtlichen Figuren 4A bis 6B ist das Extensionsanschlagselement im obersten Extensionsbegrenzungsloch 12, 12a eingesetzt, das mit "0"° gekennzeichnet ist. Das Flexionsanschlagselement 37 ist jeweils in einem Flexionsbegrenzungsloch 13, 13a eingesetzt, so daß eine maximale Flexion des Unterschenkels gegenüber dem Oberschenkel von 60° zugelassen wird. Das Führungs- und Feststellelement 31 besteht jeweils aus einer Schraube mit verbreitertem, das Langloch 22 der Gegenanschlagverstellelementhälfte 18b überdeckenden Schraubenkopf und einer auf der gegenüberliegenden Seite an der Gegenanschlagerstellelementhälfte 18a anliegenden Mutter, um die beiden Gegenanschlagverstellelementhälften 18a, 18b zusammenzuspannen und gegen die dazwischenliegende distale Schiene 1 zu drücken. In festgeschraubtem Zustand ist somit das Gegenanschlagverstellelement 18 nicht mehr relativ zur distalen Schiene 1 verschwenkbar. Wird das Führungs- und Feststellelement 31 dagegen gelockert, läßt es sich längs der Langlöcher 22, 30 verschieben, wodurch ein Verschwenken des Gegenanschlagverstellelements 18 relativ zur distalen Schiene 1 in einem Winkelbereich von 0° bis -15° ermöglicht wird. Dieser Relativwinkel kann anhand einer Skala 38 abgelesen werden.

In den Figuren 4A und 4B ist das Gegenanschlagverstellelement 18 derart an der distalen Schiene 1 festgelegt, daß der Relativwinkel zwischen diesen Teilen 0° beträgt. Befindet sich die distale Schiene 1 in dargestelltem maximalen Extensionszustand bezüglich der proximalen Schiene 3, schlägt der Gegenanschlag 25 des Gegenanschlagverstellelements 18 am Extensionsanschlagselement 26 an, das an der proximalen Schiene 3 und der mit dieser fest verbundenen Gewindescheibe 33 befestigt ist. Dieser Zustand ist auch aus der perspektivischen Darstellung von Figur 2 ersichtlich.

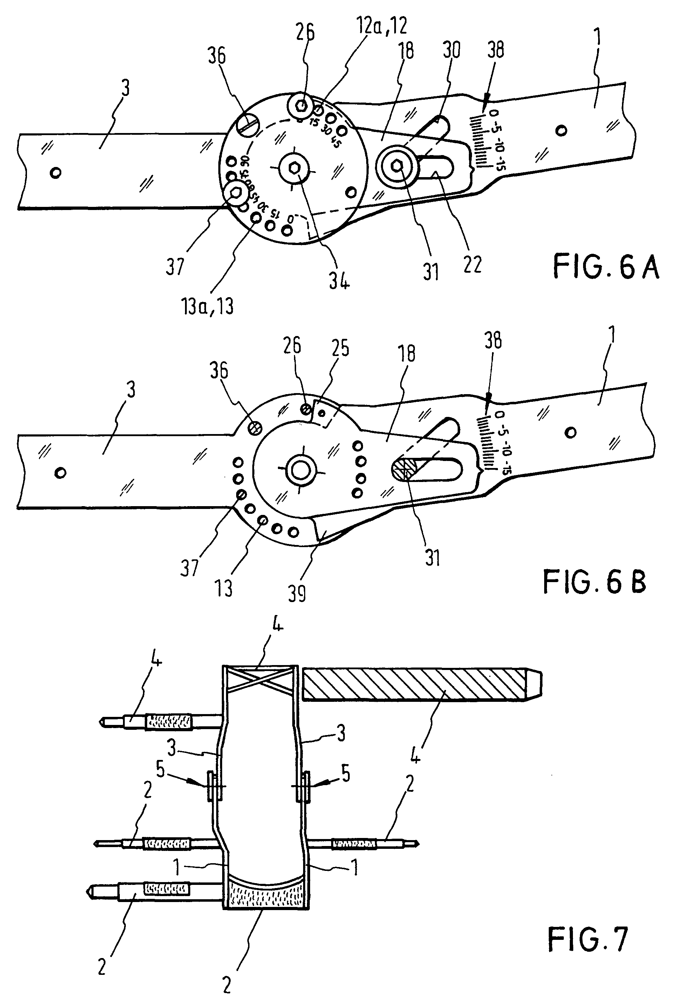

Von dieser Lage aus läßt sich die distale Schiene 1 um einen Winkel von 60° in Flexionsrichtung frei verschwenken, bis ein Flexionsgegenanschlag 39, der an der distalen Schiene 1 ausgebildet ist, am Flexionsanschlagelement 37 der proximalen Schiene 3 anschlägt. Diese Lage ist in den Figuren 5A und 5B dargestellt.

Wie erkennbar, läßt sich somit durch das Einsetzen des Extensionsanschlagelements 26 in die entsprechenden Extensionsbegrenzungslöcher 12 der maximale Extensionsbereich auf 0°, 15°, 30° bzw. 45° begrenzen. Gleichzeitig läßt sich durch Einsetzen des Flexionsanschlagelements 37 in die verschiedenen Flexionsbegrenzungslöcher 13 eine Flexionsbegrenzung zwischen 0° und 90°, abgestuft in 15°-Schritten, einstellen.

Wie aus den Figuren 6A und 6B ersichtlich, läßt sich aufgrund des erfindungsgemäßen Gegenanschlagverstellelements 18 zusätzlich zu dieser gestuften Einstellung des Extensionsbegrenzungswinkels eine weitere stufenlose Verschwenkung der distalen Schiene 1 gegenüber der proximalen Schiene 3 in einem Bereich von bis zu 15° in Extensionsrichtung bewirken. Dies erfolgt durch Lösen des Führungs- und Feststellelements 31, so daß auch in dem Fall, daß der Gegenanschlag 25 am Extensionsanschlagelement 26 anliegt, eine weitere Relativbewegung zwischen der distalen Schiene 1 und dem Gegenabschlagverstellelement 18 bis zu 15° in Extensionsrichtung möglich ist. Hierbei bewegt sich das Führungs- und Feststellelement 31 längs der Langlöcher 22, 30 in Richtung zur Schwenkachse 9 bzw. schräg nach unten. Wird, wie in den Figuren 6A und 6B gezeigt, eine Extensionsbegrenzung in einem Überstreckungsbereich von -15° gewünscht, wird das Führungs- und Feststellelement 31 in der -15°-Stellung an der distalen Schiene 1 festgeschraubt und das Extensionsanschlagselement 26 in das 0°-Extensionsbegrenzungsloch 12 der proximalen Schiene 3 eingesetzt.

Um beim Festschrauben des Führungs- und Feststellelements 31 ein unerwünschtes Mitdrehen der Mutter zu vermeiden, kann diese einen Ansatz mit parallelen Seitenwänden aufweisen, welche mit geringem Spiel innerhalb des Langlochs 30 der distalen Schiene 1 geführt sind.

Zur Veranschaulichung der Gesamtanordnung wird auf Figur 7 verwiesen, welche eine Vorderansicht der erfindungsgemäßen Gelenkstütze in schematischer Darstellung zeigt. Wie ersichtlich, können die Befestigungsabschnitte la, 3a der distalen Schiene 1 bzw. der proximalen Schiene 3 in geeigneter Weise abgewinkelt oder gekröpft sein, um eine optimale Anpassung im Gelenkbereich zu erhalten. Zur Befestigung der Schienen am Unter- bzw. Oberschenkel dienen bekannte Gurte 2 bzw. 4.

Obwohl die erfindungsgemäße Gelenkstütze anhand einer Knieorthese näher beschrieben wurde, ist es ohne weiteres möglich, eine derartige Gelenkstütze auch anderweitig, beispielsweise im Bereich des Ellbogens oder - in verkleinerter Ausführung - für Fingergelenke, einzusetzen. Weiterhin ist es auch ohne weiteres möglich, den Anschlag nicht nur in Extensionsrichtung über einen bestimmten Winkelbereich stufenlos veränderbar bezüglich der distalen Schiene 1 zu positionieren, sondern auch den Anschlag in Flexionsrichtung. Weiterhin ist es auch möglich, das Gegenanschlagverstellelement 18 nicht an der distalen Schiene 1, sondern an der proximalen Schiene 3 stufenlos verstellbar anzubringen.

## Patentansprüche

1. Gelenkstütze, insbesondere Kniegelenkstütze, zur Verhinderung einer Torsion eines distalen Körperglieds gegenüber einem damit gelenkig verbundenen proximalen Körperglied, mit am distalen bzw. proximalen Körperglied befestigbaren, mittels eines Drehgelenks (5) verbundenen Schienen (1, 3), wobei der freie Schwenkbereich der distalen Schiene (1) relativ zur proximalen Schiene (3) mittels eines Anschlagelements (26) begrenzbar ist, das an einer (3) der Schienen (1, 3) befestigbar ist und mit einem Gegenanschlag (25) der anderen Schiene (1) zusammenwirkt, wobei der Gegenanschlag (25) an einem um die Schwenkachse (9) des Kniegelenks (8) herum drehbeweglich gelagerten Gegenanschlagverstellelement (18) vorgesehen ist, **dadurch gekennzeichnet,** daß das Anschlagelement (26) in vorbestimmten, unterschiedlichen Positionen an einem schwenkachsennahen Gelenkabschnitt (3b) der einen Schiene (3) befestigbar ist und das Gegenanschlagverstellelement (18) aus einem Schwenkhebel besteht, der im Bereich eines sich über den schwenkachsennahen Gelenkabschnitt (3b) hinaus erstreckenden Handhabungsabschnitts (21) mittels eines innerhalb einer Langlocheinrichtung (22, 30) verschiebbaren Führungs- und Feststellelements (31) an der dem Gegenanschlagverstellelement (18) zugeordneten Schiene (1) festlegbar ist.

2. Gelenkstütze nach Anspruch 1, **dadurch gekennzeichnet,** daß der Handhabungsabschnitt (21) des Gegenanschlagverstellelements (18) und die diesem zugeordnete Schiene (1) jeweils ein Langloch (22 bzw. 30) aufweisen, deren Längsachsen sich in einem bestimmten Winkel schneiden, und daß das Führungs- und Feststellelement (31) durch die beiden Langlöcher (22, 30) in deren Überschneidungsbereich hindurchgeführt ist, so daß eine Verschiebung des Führungs- und Feststellelements (31) zwangsweise eine Relativbewegung zwischen dem Gegenanschlagverstellelement (18) und der Schiene (1) bewirkt.

3. Gelenkstütze nach Anspruch 2, **dadurch gekennzeichnet,** daß das am Gegenanschlagverstellelement (18) vorgesehene Langloch (22) in Richtung der Längsachse des Handhabungsabschnitts (21) verläuft und das an der zugeordneten Schiene (1) vorgesehene Langloch (30) mit der Längsachse der Schiene (1) einen Winkel von 20° bis 60°, insbesondere 35°, einschließt.

4. Gelenkstütze nach einem der vorhergehenden Ansprüche, **dadurch gekennzeichnet,** daß das Gegenanschlagverstellelement (18) aus mindestens einem Plattenelement aus Stahlblech besteht.

5. Gelenkstütze nach einem der vorhergehenden Ansprüche, **dadurch gekennzeichnet,** daß das Gegenanschlagverstellelement (18) aus zwei zumindest im wesentlichen identischen Hälften (18a, 18b) besteht, die beidseits der zugeordneten Schiene (1) angeordnet sind.

6. Gelenkstütze nach einem der vorhergehenden Ansprüche, **dadurch gekennzeichnet,** daß der Gegenanschlag (25) am Gegenanschlagverstellelement (18) in Form einer vorspringenden Nase ausgebildet ist.

7. Gelenkstütze nach einem der vorhergehenden Ansprüche, **dadurch gekennzeichnet,** daß das Führungs- und Feststellelement (31) als Klemmschraube ausgebildet ist.

8. Gelenkstütze nach einem der vorhergehenden Ansprüche, **dadurch gekennzeichnet,** daß der Verstellbereich der zugeordneten Schiene (1) gegenüber dem Gegenanschlagverstellelement (18) 0° bis -20°, insbesondere 0° bis -15°, in Extensionsrichtung, d.h. in Streckrichtung der Körperglieder, bezüglich der durch das Extensionsanschlagelement (26) festgelegten Winkelposition beträgt.

## Claims

1. Joint brace, in particular knee-joint brace, for preventing twisting of a distal body part relative to a proximal body part connected to the latter in an articulated manner, with bars (1, 3) which can be secured on the distal and proximal body part, respectively, and are connected by means of a hinge (5), the free pivoting range of the distal bar (1) relative to the proximal bar (3) being able to be limited by means of a stop element (26) which can be secured on one (3) of the bars (1, 3) and cooperates with a counterstop (25) on the other bar (1), the counterstop (25) being provided on a counterstop adjustment element (18) which is mounted such that it can be rotated about the pivot axis (9) of the knee joint (8), characterized in that the stop element (26) can be secured in predetermined different positions on a joint portion (3b) of one bar (3) near the pivot axis, and the counterstop adjustment element (18) consists of a pivot lever which, in the area of a manoeuvring portion (21) extending beyond the joint portion (3b) near the pivot axis, can be fixed on the bar (1) associated with the counterstop adjustment element (18) by means of a guiding and fixing element (31) which can be displaced inside an oblong hole device (22, 30).

2. Joint brace according to Claim 1, characterized in that the manoeuvring portion (21) of the counterstop adjustment element (18) and the bar (1) associated with it each have an oblong hole (22, 30, respectively), the longitudinal axes of these holes intersecting at a defined angle, and in that the guiding and fixing element (31) is guided through the two oblong holes (22, 30) in their intersection area, so that a displacement of the guiding and fixing element (31) necessarily effects a relative movement between the counterstop adjustment element (18) and the bar (1).

3. Joint brace according to Claim 2, characterized in that the oblong hole (22) provided on the counterstop adjustment element (18) extends in the direction of the longitudinal axis of the manoeuvring portion (21), and the oblong hole (30) provided on the associated bar (1) encloses with the longitudinal axis of the bar (1) an angle of 20° to 60°, in particular 35°.

4. Joint brace according to one of the preceding claims, characterized in that the counterstop adjustment element (18) consists of at least one plate element made of sheet steel.

5. Joint brace according to one of the preceding claims, characterized in that the counterstop adjustment element (18) consists of two at least substantially identical halves (18a, 18b) which are arranged on both sides of the associated bar (1).

6. Joint brace according to one of the preceding claims, characterized in that the counterstop (25) on the counterstop adjustment element (18) is designed in the form of a protruding nose.

7. Joint brace according to one of the preceding claims, characterized in that the guiding and fixing element (31) is designed as a clamping screw.

8. Joint brace according to one of the preceding claims, characterized in that the adjustment range of the associated bar (1) relative to the counterstop adjustment element (18) is 0° to -20°, in particular 0° to -15°, in the extension direction, i.e. in the direction of extension of the body parts, with respect to the angle position fixed by the extension stop element (26).

## Revendications

1. Support d'articulation, en particulier pour le genou, destiné à empêcher une torsion d'un membre corporel distal par rapport à un membre corporel proximal relié de façon articulée à celui-ci, au moyen de tringles (1, 3) susceptibles d'être fixées au membre corporel distal et au membre corporel proximal, et reliées au moyen d'une articulation rotative (5), dans lequel la plage de pivotement libre des tringles distales (1) par rapport aux tringles proximales (3) peut être limitée au moyen d'un élément de butée (26), celui-ci pouvant être fixé sur l'une des tringles (1, 3) et coopérant avec une contrebutée (25) de l'autre tringle (1), ladite contrebutée (25) étant prévue sur un élément de réglage (18) monté avec faculté de rotation autour de l'axe de pivotement (9) de l'articulation du genou (8),
caractérisé en ce que l'élément de butée (26) est susceptible d'être fixé à des positions prédéterminées différentes sur un tronçon d'articulation (3b) proche de l'axe de pivotement et appartenant à l'une des tringles (3), et l'élément de réglage (18) est constitué par un levier pivotant qui peut être fixé, dans la région d'un tronçon de manipulation (21) qui s'étend au-delà du tronçon d'articulation (3b) proche de l'axe de pivotement, sur le rail (1) associé à l'élément de réglage (18) au moyen d'un élément de guidage et de fixation (31) capable de translation à l'intérieur d'un agencement à trou oblong (22, 30).

2. Support d'articulation selon la revendication 1, caractérisé en ce que le tronçon de manipulation (21) de l'élément de réglage (18) et le rail (1) qui lui est associé présentent chacun un trou oblong (22, respectivement 30), dont les axes longitudinaux se recoupent sous un certain angle, et en ce que l'élément de guidage et de fixation (31) est passé à travers les deux trous oblongs (22, 30) dans leur zone de recoupement, de sorte qu'une translation de l'élément de guidage et de fixation (31) provoque obligatoirement un mouvement relatif entre l'élément de réglage (18) et le rail (1).

3. Support d'articulation selon la revendication 2, caractérisé en ce que le trou oblong (22) prévu sur l'élément de réglage (18) s'étend en direction de l'axe du tronçon de manipulation (21), et en ce que le trou oblong (30) prévu sur le rail associé (1) forme avec l'axe longitudinal du rail (1) un angle de 20 à 60°, en particulier de 35°.

4. Support d'articulation selon l'une des revendications précédentes, caractérisé en ce que l'élément de réglage (18) est constitué par au moins un élément en forme de plaquette en tôle d'acier.

5. Support d'articulation selon l'une des revendications précédentes, caractérisé en ce que l'élément de réglage (18) est constitué en deux moitiés au moins sensiblement identiques (18a,18 b) qui sont agencées des deux côtés du rail associé (1).

6. Support d'articulation selon l'une des revendications précédentes, caractérisé en ce que la contrebutée (25) est réalisée sur l'élément de réglage (18) sous forme d'un bec en saillie.

7. Support d'articulation selon l'une des revendications précédentes, caractérisé en ce que l'élément de guidage et de fixation (31) est réalisé sous forme de vis de serrage.

8. Support d'articulation selon l'une des revendications précédentes, caractérisé en ce que la zone de réglage du rail associé (1) par rapport à l'élément de réglage (18) s'élève à 0 à - 20°, en particulier 0 à -15°, dans la direction d'extension, c'est-à-dire dans la direction d'extension du membre corporel, rapport à la position angulaire fixée par l'élément de butée (26) en extension.
